# EUROPEAN PATENT SPECIFICATION

(11) **EP 1 824 543 B1**
(45) Date of publication and mention of the grant of the patent: **23.01.2013**
(21) Application number: 05825913.6
(22) Date of filing: 07.12.2005
(51) Int. Cl.: B65D 83/54, B65D 83/14, A61M 15/00

(54) **ELASTOMER SEALS FOR USE IN MEDICINAL AEROSOL DEVICES**
ELASTOMERE DICHTUNGEN ZUR VERWENDUNG IN MEDIZINISCHEN AEROSOLVORRICHTUNGEN
JOINTS D'ETANCHEITE EN ELASTOMERE UTILISABLES DANS DES DISPOSITIFS AEROSOLS POUR MEDICAMENTS

(30) Priority: 15.12.2004 US 636177 P
(43) Date of publication of application: 29.08.2007
(73) Proprietor: 3M Innovative Properties Company, St. Paul, MN 55133-3427 (US)
(72) Inventor: FENN, Percy T., Saint Paul, MN 55133-3427 (US); WINKER, Theodore A., Saint Paul, MN 55133-3427 (US); ADAIR, Eric, W., Saint Paul, MN 55133-3427 (US)
(74) Representative: Vossius & Partner
(86) International application number: PCT/US2005/044149
(87) International publication number: WO 2006/065588

(56) References cited:
- WO-A1-02/24548
- WO-A1-95/02633
- WO-A1-03/078516
- WO-A1-2005/028547
- US-A- 5 252 401
- US-A- 5 788 216
- US-A1- 2003 138 559
- US-A1- 2004 044 139
- US-A1- 2004 211 411
- US-A1- 2004 223 916
- US-B2- 6 750 210
- US-B2- 6 887 927

## Description

### Field

The present invention relates to seals for use in medicinal aerosol devices, in particular valves for medicinal aerosol devices, such as metered dose inhalers.

### Background

Medicinal aerosol devices are commonly used to deliver aerosolized medicaments to patients, such as, for example delivering medicament to the lung or to the nasal passages. Typical medicinal aerosol devices include metered dose inhalers, nebulizers, dry powder inhalers, and nasal sprays. A typical device comprises a medicinal composition held within a container that is equipped with a valve. The valve allows for a controlled release of medicament that may be delivered to the patient. The valve generally requires one or more elastomer seals to prevent unintended leakage of the medicinal composition during storage and/or use. In some cases the medicinal composition is held under pressure and the elastomer seals need to be able to withstand this pressure.

In the context of pressurized metered dose inhalers for oral or nasal inhalation, particularly those containing hydrofluoroalkane propellants HFA-134a and/or HFA 227, valve seals are considered a critical performance component. A number of materials have been previously used or proposed for use as elastomer seals in medicinal aerosol devices, including, for example, butyl rubber, butadiene-acrylonitrile rubber, neoprene, nitrile rubber, olefinic thermoplastic elastomers, fluoropolymers, ethylene-propylene-diene (EPDM) rubber, and ethylene-propylene (EPM) rubber. There is still a need, however, for improved seals over the currently used materials. Among the desired properties of a sealing material are: resistance to excessive swelling when in contact with a medicinal composition, ability to provide a low leakage rate when used in an MDI, ability to allow reciprocal movement of a valve stem without sticking or necessitating excessively high forces to allow movement of the valve stem (i.e., acceptable force to fire), and having low levels of extractable material.

US 2004 022 39 16 discloses a canister for a metered dose inhaler having a metering valve in the form of a slide valve in which the open/close mechanism comprises a sealing ring. The canister has part or all of its internal surfaces coated with one or more fluorocarbon polymers, including hydrofluoroalkane fluorocarbon propellants.

### Summary of the Invention

It has now been found that crosslinked perfluorinated elastomers are excellent seal materials for medicinal aerosol devices. This is surprising because conventional fluoroelastomers such as Viton™ (Dupont Dow), which are not perfluorinated, can have excessive swelling in HFA propellants.

The present invention thus provides medicinal aerosol device according to claim 1.

The invention will be further understood by those skilled in the art upon consideration of the remainder of the disclosure, including the Detailed Description and the appended claims.

### Brief Description of the Drawings

Preferred embodiments of the invention will now be described in greater detail below with reference to the attached drawings, wherein:
FIG. 1 is a partial cross-sectional view of one embodiment of a device of the invention, wherein the valve stem is in the extended closed position.
FIG. 2 is a partial cross-sectional view of the embodiment illustrated in FIG. 1, wherein the valve stem is in the compressed open position.

### Detailed Description

The term crosslinked elastomer is used to indicate a polymeric material that can recover most or all of its original dimensions after undergoing extension or compression (i.e., a material exhibiting rubber-like elasticity). Crosslinking may comprise chemical bonds between neighboring polymer chains (also generically referred to as vulcanization) or it may comprise physical crosslinks provided by crystallization or phase separation of hard segments in segmented or graft copolymers. Examples of conventional chemically crosslinked elastomers include butyl rubber, butadiene-acrylonitrile rubber, neoprene, nitrile rubber, and ethylene-propylene-diene (EPDM) rubber. Examples of conventional physically crosslinked elastomers include styrene-isoprene block copolymers.

Crosslinked perfluorinated elastomer sealing members of the present invention may be characterized according to various tests used to characterize rubbery materials. For instance, Shore A hardness of a sealing member for use in the invention is often between about 50 and about 90, and more commonly between about 70 and about 85.

Crosslinked perfluorinated elastomer sealing members of the present invention may also be characterized by resistance to long-term flow or creep. Although certain uncrosslinked elastomers may exhibit rubber-like behavior over relatively short time periods, they are susceptible to long term flow or creep. Sealing members of the present invention preferably have a suitable compression set in order that the seal(s) between the sealing member and the other components of the device remains adequate over the life of the device. Compression set tests measure the amount of non-recoverable deformation in an elastomeric material after application of a constant force over a fixed time period at a constant temperature. This may be measured according to the American Society for Testing Materials (ASTM) standard test method D395-97. Briefly, a test sample of a fixed size is placed between two plates and compressed with a fixed force over a fixed time period (typically 22 hours) at a constant temperature (typically 70 °C). The thickness of the test sample is measured both before and after compression. The compression set is the difference between the original thickness and the final thickness of the test sample and is reported as a relative percentage of the original total thickness. The compression set value tested according to ASTM D395 Test Method A (conditions of 22 hours and 70 °C), Compression Set Under Constant Force in Air, may be less than about 40%, often less than about 30%, and sometimes less than about 20%.

The term 'perfluoro' or 'perfluorinated' in connection with this invention is used to mean that the respective compound has virtually all hydrogen atoms replaced by fluorine atoms without however excluding the possibility that some of the hydrogen atoms have been replaced with chlorine, bromine or iodine atoms. Specifically, the term 'perfluorinated elastomer' is intended to mean a fluoroelastomer that has a perfluorinated backbone, i.e. a backbone in which the hydrogen atoms are replaced with fluorine atoms without excluding polymers wherein some of the hydrogen atoms have been replaced with another halogen than fluorine such as for example chlorine as may be the case if the fluoropolymer derives from a polymerisation involving chlorotrifluoroethylene. Perfluorinated compounds include those designated FFKM according to ASTM D1418-01a. Suitable examples of perfluoroelastomers include polymers derived from perfluoro(alkylvinyl) ethers and perfluoro(alkoxyvinyl) ethers and mixtures thereof. Copolymers of tetrafluoroethylene and perfluoro(alklvinyl) ethers and/or perfluoro(alkoxyvinyl) ethers are also suitable, including poly[tetrafluoroethylene-coperfluoro(methyl vinyl ether)], also referred to below as TFE-PMVE copolymer. Fluoroelastomers and in particular perfluoroelastomers are described in more detail in "Modem Fluoropolymers", edited by John Scheirs, Wiley Science 1997. Fluoroelastomers are elastomers that may be prepared by curing a fluoroelastomer precursor ("fluoroelastomer gum") made from monomers containing one or more atoms of fluorine, or copolymers of such monomers with other monomers, the fluoromonomer(s) being present in the greatest amount by mass. The fluoroelastomer precursor is a fluoropolymer that is suitable to prepare a fluoroelastomer having desired elasticity properties. Typically, the fluoroelastomer precursor is an amorphous fluoropolymer or a fluoropolymer that hardly shows a melting point. When the fluoropolymer has a perfluorinated backbone, a perfluoroelastomer results.

Crosslinked perfluorinated elastomers are typically prepared by formulating a fluorocarbon elastomer gum containing a cure system with cure additives and curing to yield a crosslinked elastomer. Other additives, such as fillers and metallic oxides may also be added to adjust the physical or chemical properties of the elastomer. Such polymers are often prepared by high pressure, free-radical, aqueous emulsion polymerization.

Sealing members comprising a crosslinked perfluorinated elastomer may further comprise other additives such as carbon black, stabilizers, plasticizers, lubricants, fillers, and processing aids typically utilized in fluoropolymer compounding.

Fluoropolymer fillers may also be present in the sealing members. Generally, from 1 to 50 parts filler per hundred parts fluoroelastomer of fluoropolymer filler is used. In one embodiment, the fluoropolymer filler can be finely divided and easily dispersed as a solid at the highest temperature used in fabrication and curing of the sealing member. By solid, it is meant that the filler material, if partially crystalline, will have a crystalline melting temperature above the processing temperature(s) of a curable sealing member. A preferred way to incorporate fluoropolymer filler is by blending latices. This procedure, including various kinds of fluoropolymer filler, is described in United States Patent No. 6,720,360 (U.S. Ser. No. 09/495,600, filed Feb. 1, 2000).

One or more acid acceptors can also be added to the formulations. Commonly used acid acceptors include, for example, zinc oxide, calcium hydroxide, calcium carbonate, and magnesium oxide.

Curable fluoroelastomer compositions can be prepared by mixing a fluoroelastomer containing a cure system with cure additives, a catalyst, additional optional curative(s), any optional adjuvants, and any other optional additive(s), in conventional rubber processing equipment. The desired amounts of compounding ingredients and other adjuvants or ingredients can be added to the unvulcanized fluorocarbon gum stock and intimately admixed or compounded therewith by employing any of the usual rubber mixing devices such as internal mixers, (e.g., Banbury mixers), roll mills, or any other convenient mixing device. It may be desired to keep the temperature of the mixture during the mixing process from rising above about 120 °C. It may be desired during mixing to distribute the components and adjuvants uniformly throughout the gum for effective cure.

The mixture may then be processed and shaped, such as by extrusion (for example, in the shape of a flat seal) or by molding (for example, in the form of an O-ring seal). The shaped article may then be heated to cure the gum composition and form a cured article. Pressing of the compounded mixture (i.e., press cure) is often conducted at a temperature sufficient to cure the mixture in a desired time duration under a suitable pressure. Generally, this is between about 95 °C and about 230 °C, preferably between about 150 °C and about 205 °C, for a period of from about 1 minute to 15 hours, typically from 5 minutes to 30 minutes. A pressure of between about 700 kPa and about 20,600 kPa is usually imposed on the compounded mixture in a mold. The molds first may be coated with a release agent and prebaked. The molded vulcanizate is often post-cured (e.g., ovencured) at a temperature and for a time sufficient to complete the curing, usually between about 150 °C and about 300 °C, typically at about 232 °C, for a period of from about 2 hours to 50 hours or more, generally increasing with the cross-sectional thickness of the article. For thick sections, the temperature during the post cure is often raised gradually from the lower limit of the range to the desired maximum temperature. The maximum temperature used is preferably about 300 °C., and this value may be held for about 4 hours or more. Following cure, the article may be heat aged in air. One useful example of a heat aging protocol ages the article in air for about 70 hours at a temperature of about 290 °C.

The fluoropolymer compositions are useful in production of sealing members, such as O-rings, diaphragms, and gaskets, for use in medicinal aerosol devices. In one embodiment, such articles may be produced by molding a compounded formulation of the fluoropolymer composition with various additives under pressure, curing the article, and then subjecting it to a post-cure cycle. In another embodiment, such articles may be produced by preparing a cured sheet of material that is subsequently punched, cut, or shaped into the desired article.

Further detail regarding preparation, formulation, and compounding of crosslinked perfluorinated elastomers may be found in U. S. Patents Nos. 4,948,853, 5,260,351, 6,657,012, 6,657,013, 6,730,760, and 6,794,457, U. S. Published Patent Application Nos. 2002/00177666, 2002/0145228, 2002/0183458, 2004/0044139, US2004/0072959, and PCT Publication No. WO 99/4893.

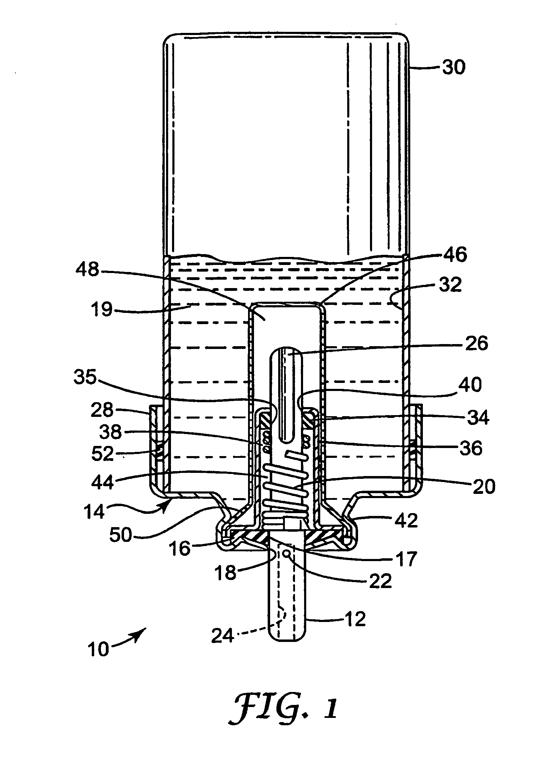

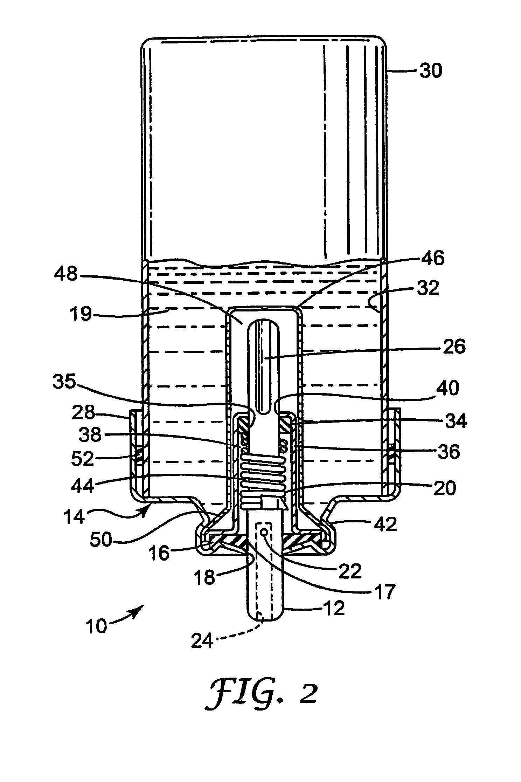

One embodiment of the device of the invention will be described with reference to FIGS. 1 and 2. FIG. 1 shows device 10 comprising valve stem 12, casing member 14, and diaphragm 16. The casing member has walls defining casing aperture 18, and the diaphragm has walls defining diaphragm aperture 17. The valve stem passes through and is in slidable sealing engagement with the diaphragm aperture. The diaphragm is also in sealing engagement with casing member 14. Diaphragm 16 comprises a crosslinked perfluorinated elastomer sealing member. Such a sealing member can be one piece or it can be in the form of a plurality of thinner layers arranged in a stack.

The illustrated embodiment is a device for use with pharmaceutical formulations. The diaphragm in the illustrated embodiment is a single piece of a thickness sufficient to form an effective seal with the casing member, preferably about 0.125 mm (0.005 inch) to about 1.25 mm (0.050 inch). It has an outside diameter of about 8.6 mm (0.340 inch), and an inside diameter sufficient to form an effective seal with the valve stem. As valve stems having an outside diameter of about 2.79 mm (0.110 inch) are commonly used, suitable diaphragm inside diameter can be in the range of about 2.03 mm (0.080 inch) to about 2.67 mm (0.105 inch). Diaphragm dimensions suitable for use with other general types of devices can be easily selected by those skilled in the art.

Valve stem 12 is in slidable engagement with diaphragm aperture 17. Helical spring 20 holds the valve stem in an extended closed position as illustrated in FIG. 1. Valve stem 12 has walls defining orifice 22 which communicates with exit chamber 24 in the valve stem. The valve stem also has walls defining channel 26.

In the illustrated embodiment casing member 14 comprises mounting cup 28 and canister body 30 and defines formulation chamber 32. The illustrated embodiment further comprises tank seal 34 having walls defining tank seal aperture 35, and metering tank 36 having inlet end 38, inlet aperture 40, and outlet end 42. The metering tank also has walls defining metering chamber 44 of predetermined volume (e.g., 50 µL). Outlet end 42 of metering tank 36 is in sealing engagement with diaphragm 16, and valve stem 12 passes through inlet aperture 40 and is in slidable engagement with tank seal 34. The tank seal 34 comprises a crosslinked perfluorinated elastomer sealing member.

When device 10 is intended for use with a suspension aerosol formulation it may further comprise a retaining cup 46 fixed to mounting cup 28 and having walls defining retention chamber 48 and aperture 50. When intended for use with a solution aerosol formulation retaining cup 46 is optional. Also illustrated in device 10 is sealing member 52 in the form of an O-ring that substantially seals formulation chamber 32 defined by mounting cup 28 and canister body 30. Sealing member 52 preferably comprises the elastomer described above.

Operation of device 10 is illustrated in FIGS. 1 and 2. In FIG. 1, the device is in the extended closed position. Aperture 50 allows open communication between retention chamber 48 and formulation chamber 32, thus allowing the aerosol formulation to enter the retention chamber. Channel 26 allows open communication between the retention chamber and metering chamber 44 thus allowing a predetermined amount of aerosol formulation to enter the metering chamber through inlet aperture 40. Diaphragm 16 seals outlet end 42 of the metering tank.

FIG. 2 shows device 10 in the compressed open position. As valve stem 12 is depressed channel 26 is moved relative to tank seal 34 such that inlet aperture 40 and tank seal aperture 35 are substantially sealed, thus isolating a metered dose of formulation within metering chamber 44. Further depression of the valve stem causes orifice 22 to pass through aperture 18 and into the metering chamber, whereupon the metered dose is exposed to ambient pressure. Rapid vaporization of the propellant causes the metered dose to be forced through the orifice, and into and through exit chamber 24. Device 10 is commonly used in combination with an actuator that facilitates inhalation of the resulting aerosol by a patient

One embodiment of the device of the present invention is a metered dose configuration substantially as described above and illustrated in FIGS. 1 and 2. Other particular configurations, metered dose or otherwise, are well known to those skilled in the art and suitable. For example the devices described in U.S. Pat. Nos. 4,819,834 (Thiel), 4,407,481 (Bolton), 3,052,382 (Gawthrop), 3,049,269 (Gawthrop), 2,980,301 (DeGorter), 2,968,427 (Meshberg), 2,892,576 (Ward), 2,886,217 (Thiel), and 2,721,010 (Meshberg) involve a valve stem, a diaphragm, and a casing member in the general relationship described herein. Generally any and all sealing members (such as diaphragms, seals, and gaskets) that serve to minimize and/or prevent escape of components, especially propellant, from such assemblies can comprise the above described elastomer.

In the embodiment shown in FIGS. 1 and 2, the device comprises three distinct sealing members, namely diaphragm 16, tank seal 34, and O-ring 52, at least one of which comprises a crosslinked perfluorinated elastomer sealing member. Additional sealing members may also be included in the device, for example, a ferrule gasket such as that described in U. S. Patent No. 5,775,321 (Alband). Conventional sealing members may also be used for one or more of the sealing members in a device. For example, in the embodiment described above, the device may have an O-ring 52 comprising a conventional sealing member used along with a crosslinked perfluorinated elastomer diaphragm 16 and/or tank seal 34. Examples of suitable conventional sealing materials include ethylene-propylene-diene (EPDM) rubber, ethylene-propylene (EPM) rubber, butyl rubber, neoprene, butadiene-acrylonitrile (or "Buna") rubber, styrene-ethylene/butylene-styrene block copolymers, copolymers of ethylene and either butene, hexene, or octene as disclosed in U. S. Patent No. 5,290,539 (Marecki) or mixtures of the foregoing. Conventional sealing materials may be used in conjunction with the crosslinked perfluorinated elastomer materials of the present invention to form a single sealing member, for example, by combining a layer of crosslinked perfluorinated elastomer with a layer of a conventional sealing material. An example of such a multiple layer sealing member is disclosed in U.S. Patent Application No. 10/878783 (Winker et al.).

Crosslinked perfluorinated elastomer sealing members of the present invention are also suitable for use in other metered dose devices comprising a medicinal composition, such as those disclosed in U. S. Patent Nos. 5,772,085 (Bryant et al.), 6,454,140 (Jinks), 6,644,517 (Thiel et al.), 6,640,805 (Castro et al.), U.S. Published Patent Applications Nos. 2003/010794 (Herdtle et al.), 2003/127464 (Bryant et al.), 2003/121935 (Arsenault et al.), 2004/139965 (Greenleaf et al.), and 2004/139966 (Hodson).

Examples of suitable propellants for use in aerosol formulations of the present invention include 1,1,1,2-tetrafluoroethane (HFC-134a), 1,1,1,2,3,3,3-heptafluoropropane (HFC-227), fluorotrichloromethane, dichlorodifluoromethane, and 1,2-dichlorotetrafluoroethane, and mixtures thereof. Preferred propellants are 1,1,1,2-tetrafluoroethane (HFC-134a), 1,1,1,2,3,3,3-heptafluoropropane (HFC-227), and mixtures thereof.

Preferred medicinal compositions generally comprise HFC- 134a, HFC-227, or a mixture thereof in an amount effective to function as an aerosol propellant, a drug having local or systemic action and suitable for use by inhalation, and any optional formulation excipients. In a preferred embodiment, medicinal compositions of the present invention comprise from 1 to 25% ethanol by weight of the total formulation.

As used herein, the term "drug," includes its equivalents, "bioactive agent," and "medicament" and is intended to have its broadest meaning as including substances intended for use in the diagnosis, cure, mitigation, treatment or prevention of disease, or to affect the structure or function of the body. The drugs can be neutral or ionic. Preferably, they are suitable for oral and/or nasal inhalation. Delivery to the respiratory tract and/or lung, in order to effect bronchodilation and to treat conditions such as asthma and chronic obstructive pulmonary disease, is preferably by oral inhalation. Alternatively, to treat conditions such as rhinitis or allergic rhinitis, delivery is preferably by nasal inhalation. Preferred drugs are asthma, allergy, or chronic obstructive pulmonary disease medications.

Suitable drugs include, for example, antiallergics, anticancer agents, antifungals, antineoplastic agents, analgesics, bronchodilators, antihistamines, antiviral agents, antitussives, anginal preparations, antibiotics, anti-inflammatories, immunomodulators, 5-lipoxygenase inhibitors, leukotriene antagonists, phospholipase A₂ inhibitors, phosphodiesterase IV inhibitors, peptides, proteins, steroids, and vaccine preparations. A group of preferred drugs include adrenaline, albuterol, atropine, beclomethasone dipropionate, budesonide, butixocort propionate, clemastine, cromolyn, epinephrine, ephedrine, fentanyl, flunisolide, fluticasone, formoterol, ipratropium bromide, isoproterenol, lidocaine, morphine, nedocromil, pentamidine isoethionate, pirbuterol, prednisolone, salmeterol, terbutaline, tetracycline, 4-amino-α,α,2-trimethyl-1H-imidazo[4,5-c]quinoline-1-ethanol, 2,5-diethyl-10-oxo-1,2,4-triazolo[1,5-c]pyrimido[5,4-b][1,4]thiazine, 1-(1-ethylpropyl)-1-hydroxy-3-phenylurea, and pharmaceutically acceptable salts and solvates thereof, and mixtures thereof. Particularly preferred drugs include pirbuterol, 4-amino-α,α,2-trimethyl-1H-imidazo[4,5-c]quinoline-1-ethanol, 2,5-diethyl-10-oxo-1,2,4-triazolo[1,5-c]pyrimido[5,4-b][1,4]thiazine, 1-(1-ethylpropyl)-1-hydroxy-3-phenylurea, and pharmaceutically acceptable salts and solvates thereof, and mixtures thereof.

The drug is present in the formulation in an amount sufficient to provide a predetermined number of therapeutically effective doses by inhalation, which can be easily determined by those skilled in the art considering the particular drug in the formulation. Optional excipients include cosolvents (e.g., ethanol, water) and surfactants (e.g., oleic acid, sorbitan esters, polyoxyethylenes, glycols) and others known to those skilled in the art.

In one embodiment, medicinal aerosol devices may be prepared by providing a container adapted to contain a medicinal aerosol formulation and equipping the container with a valve. One or more interfaces in the valve or between the container and the valve are sealed with a crosslinked perfluorinated elastomer seal. The container is filled with a medicinal composition. In one aspect the container is filled with medicinal composition prior to equipping the container with the valve. This may be done, for example, by a cold-filling process where the medicinal composition is chilled sufficiently so that it will not excessively vaporize when placed into an unsealed container. The container is then equipped with a valve and sealed, after which time the composition may be warmed to room temperature. In another aspect, the container is filled with medicinal composition subsequent to equipping the container with the valve. This may be done, for example, by a pressure-filling process where the container is equipped with a valve and the medicinal composition is subsequently introduced through the valve while being held under pressure.

In one embodiment, medicinal aerosol devices having a container equipped with a valve may be sealed by assembling the container, valve, and at least one seal such that the seal conforms to the container and/or the valve thereby sealing at least one interface in the valve or between the container and the valve with a crosslinked perfluorinated elastomer seal. The container is filled with a medicinal composition. As discussed above, the container may be filled with medicinal composition prior to or after equipping the container with the valve.

The following examples are provided to further illustrate the invention, but are not intended to limit the invention in any way. The term phr is used to indicate the number of parts of an ingredient per hundred parts of fluoroelastomer.

### Examples

### Leakage rate method

Aerosol devices were allowed to stand for at least 24 hours prior to testing. Aerosol devices were weighed individually and stored for a given time at conditions of 25 °C and 60% relative humidity. Unless otherwise specified, the devices were reweighed after 4 weeks of storage. An annual leakage rate was calculated in mg/year. Aerosol devices were identified in a way that does not contribute to the adsorption or release of moisture. Weighing was performed at room temperature. Aerosol devices to be tested were equilibrated to ambient conditions. The results shown are an average of the value determined from 6 individual devices.

### Extractables Method - Tetrahydrofuran

Crosslinked perfluorinated elastomer material (approximately 0.5 g) was cut into small pieces (approximately 1 to 2 mm in dimension) and added to an 11 dram, clear glass vial along with 10 mL of tetrahydrofuran, and subjected to ultrasound for 15-17 hours to extract material from the elastomer. After extraction, the solution was decanted to a clean, 11 dram (40.6 mL), clear glass vial and the tetrahydrofuran evaporated to leave a dried residue. The percent extractables reported is calculated by determining the mass of the residue and expressing that as a percentage of the original mass of the elastomer.

### Extractables Method - P11

Crosslinked perfluorinated elastomer material (approximately 1.2 g) was cut into small pieces (approximately 1 to 2 mm in dimension) and added to an 11 dram (40.6 mL), clear glass vial along with 10 mL of trichlorofluoromethane (Freon-11 or P11), and subjected to ultrasound for 15-17 hours to extract material from the elastomer. After extraction, the solution was decanted to a clean, 11 dram (40.6 mL), clear glass vial and the P11 evaporated to leave a dried residue. The percent extractables reported is calculated by determining the mass of the residue and expressing that as a percentage of the original mass of the elastomer.

### Swell Method

Seals were prepared and placed in a pressure cell having transparent windows. The outer diameter of the seals was measured with an optical microscope. The pressure cell was filled with a test liquid or formulation and allowed to stand at ambient conditions for a fixed period of time. The outer diameter of the seals was measured at the end of the fixed time period. Swell is reported as the increase in diameter of the seals as a percentage of the original diameter. The results reported are an average of the value determined from 3 individual seals unless otherwise indicated.

### Example 1

An approximately 0.047 inch (1.2 mm) thick sheet of crosslinked perfluorinated elastomer was prepared as follows. A perfluoroelastomer was prepared by aqueous emulsion polymerization of 65.7 mole % tetrafluoroethylene (TFE), 33.0 mole % perfluoromethyl perfluorovinyl ether (PMVE) and 1.3 mole % CF2=CFO(CF2)5CN (MV5CN) followed by blending with 30 wt % perfluoroalkoxy (PFA) copolymer. The blend was masticated on a two-roll mill for 1 to 2 minutes. Silica filler (1.5 parts per hundred parts perfluoroelastomer or phr, Aerosil® R-972, from Degussa) was then added. Dimethyl sulfone (0.5 phr) was then added. This was followed by the addition of bis-tetrabutylphosphonium perfluoroadipate (1.2 phr). Titanium dioxide (2 phr, Sakai Chemical, Osaka, Japan) was then added with a total mixing time of 15 to 20 minutes. Sample sheets approximately 0.047 inch (1.2 mm) thick were prepared by pressing the un-vulcanized compound in a mold held under pressure and temperature for 15 minutes at 370 °F (188 °C). The sheets were then subjected to a post curing cycle where the temperature was ramped from ambient to 200 °C over 45 minutes, held at 200 °C for 2 hours, increased to 250 °C over 30 minutes, held at 250 °C for 2 hours, increased to 300 °C over 30 minutes, and held at 300 °C for 4 hours before cooling to room temperature over 1 hour.

Diaphragm seals having an approximately 0.35 inch (8.9 mm) outer diameter and approximately 0.08 inch (2.0 mm) inner diameter were punched from this sheet

Devices as generally shown in Figure 1 were prepared by cold filling 15-mL aluminum aerosol vials with approximately 9 g of HFA-134A. All vials were fitted with 50 µL valves having 0.110 inch (2.79 mm) outer diameter, stainless steel valve stems. The valves were fitted with the diaphragm seals prepared above. The valves were also fitted with an O-ring seal (nitrile rubber, DB-218, American Gasket and Rubber), tank seal (nitrile rubber, DB-218, American Gasket and Rubber), and ferrule gasket (ethylene-butene copolymer, Flexomer™ DFDB 1085 NT polyolefin, Union Carbide) made from conventional sealing materials. Aerosol devices were placed in a water bath at approximately 55 °C for 3 minutes. After removal from the water bath, 5 shots were fired from each aerosol device. Leakage rates and swell results were measured according to the method described above and the results are reported in Table 1.

The amount of extractables measured according to the tetrahydrofuran extractable method was below the detection limit of the method (<0.06%). The amount of extractables measured according to the P11 extractables method was 0.8%. Extractables measured according to the tetrahydrofuran extractable method for a like device having an EPDM seal was 1.9%. Extractables measured according to the tetrahydrofuran extractable method for a like device having a nitrile rubber seal was 4.3%.

### Example 2

A device was prepared according to the general procedure of Example 1 with the exception that the aluminum aerosol vials were filled with a mixture of 9 g of 90/10 (w/w) HFA-134A and ethanol. Leakage rates and swell results are reported in Table 1.

### Example 3.

A device was prepared according to the general procedure of Example 1 with the exception that HFA-227 was used in place of HFA-134a. Leakage rates and swell results are reported in Table 1.

### Example 4

A device was prepared according to the general procedure of Example 2 with the exception that HFA-227 was used in place of HFA-134a. Leakage rates and swell results are reported in Table 1.

### Example 5

A device was prepared according to the general procedure of Example 1 with the exception that the crosslinked perfluorinated elastomer used was prepared from a perfluoroelastomer of 61.6 mole % TFE, 36.5 mole % PMVE and 1.9 mole % CF2=CFO(CF2)5CN (MV5CN) blended with 20 wt % PFA. Swell results are reported in Table 1.

### Example 6

A device was prepared according to the general procedure of Example 5 with the exception that the aluminum aerosol vials were filled with a mixture of 9 g of 90/10 (w/w) HFA-134A and ethanol. Swell results are reported in Table 1.

### Example 7

A device was prepared according to the general procedure of Example 1 with the exception that the crosslinked perfluorinated elastomer used was prepared from a perfluoroelastomer of 65.7 mole % TFE, 33.0 mole % PMVE and 1.3 mole % CF2=CFO(CF2)5CN (MV5CN) blended with 20 wt % PFA. In addition, 4 phr titanium dioxide was used. Leakage rates and swell results are reported in Table 1.

### Example 8

A device was prepared according to the general procedure of Example 7 with the exception that the aluminum aerosol vials were filled with a mixture of 9 g of 90/10 (w/w) HFA-134A and ethanol. Leakage rates and swell results are reported in Table 1.

### Example 9

A device was prepared according to the general procedure of Example 1 with the exception that the crosslinked perfluorinated elastomer used was prepared from a perfluoroelastomer of 65.7 mole % TFE, 33.0 mole % PMVE and 1.3 mole % CF2=CFO(CF2)5CN (MV5CN) which was not blended with PFA before mastication. In addition, barium sulfate (25 phr, Sakai Chemical, Osaka, Japan) was added during the addition of the titanium dioxide and 4 phr titanium dioxide was used. Swell results are reported in Table 1.

### Example 10

A device was prepared according to the general procedure of Example 9 with the exception that the aluminum aerosol vials were filled with a mixture of 9 g of 90/10 (w/w) HFA-134A and ethanol. Swell results are reported in Table 1.

### Example 11

A device was prepared according to the general procedure of Example 1 with the exception that the crosslinked perfluorinated elastomer used was prepared as follows. A perfluoroelastomer of 67.0 mole % TFE, 32.4 mole % PMVE and 0.6 mole % bromotrifluoroethylene (BTFE) was prepared by aqueous emulsion polymerization and masticated on a two-roll mill for 1 to 2 minutes. Triallyl isocyanurate (1.8 phr, Nippon Kasei,, Tokyo, Japan) was then added. This was followed by the addition of titanium dioxide (2 phr, Sakai Chemical, Osaka, Japan) and barium sulfate (25 phr, Sakai Chemical, Osaka, Japan). 2,5-dimethyl-2,5-di(tert-butylperoxy) (0.7 phr, Varox™ DBPH, R.T. Vanderbilt, Norwalk CT) was then added with a total mixing time of 15 to 20 minutes. Sample sheets approximately 0.047 inch (1.2 mm) thick were prepared by pressing the un-vulcanized compound in a mold held under pressure and temperature for 10 minutes at 350 °F (177 °C). The sheets were then subjected to a post curing cycle of 200 °C over 16 hours before cooling to room temperature over 5 minutes. Swell results are reported in Table 1.

### Example 12

A device was prepared according to the general procedure of Example 11 with the exception that the aluminum aerosol vials were filled with a mixture of 9 g of 90/10 (w/w) HFA-134A and ethanol. Swell results are reported in Table 1.

### Comparative Examples 1-4

Devices were prepared according to the general procedures of Examples 1 to 4, respectively, with the exception that a non-perfluorinated crosslinked fluoroelastomer was used in place of the perfluorinated crosslinked fluoroelastomer.

The non-perfluorinated crosslinked fluoroelastomer was prepared as follows. A fluoroelastomer of 51.2 mole % vinylidene difluoride (VDF), 24.2 mole % tetrafluoroethylene (TFE), 24.2 mole % hexafluoropropylene (HFP), and 0.4 mole % bromotrifluoroethylene (BTFE) was prepared by aqueous emulsion polymerization and blending with 20 wt % perfluoroalkoxy (PFA) copolymer. The blend was masticated on a two-roll mill for 1 to 2 minutes. Triallyl isocyanurate (2.4 phr, Nippon Kasei,, Tokyo, Japan) was then added. 2,5-dimethyl-2,5-di(tert-butylperoxy) (1.0 phr, Varox™ DBPH, R.T. Vanderbilt, Norwalk CT) was then added with a total mixing time of 10 to 15 minutes. Sample sheets approximately 0.047 inch (1.2 mm) thick were prepared by pressing the un-vulcanized compound in a mold held under pressure and temperature for 10 minutes at 350 °F (177 °C). The sheets were then subjected to a post curing cycle of 230 °C over 16 hours before cooling to room temperature over 5 minutes. Swell results are reported in Table 1.

| Table 1 | | |
|---|---|---|
| Example Number | Leak rate [mg/yr] | Swell [%] |
| 1 | 18 | 2.5 |
| 2 | 12 | 3.0 |
| 3 | 360 | 8.8 |
| 4 | 148 | 6.4 |
| 5 | - | 3.7 |
| 6 | - | 4.3 |
| 7 | 28 | 4.0 |
| 8 | 24 | 4.2 |
| 9 | - | 5.0 |
| 10 | - | 4.5 |
| 11 | - | 5.1 |
| 12 | - | 4.6 |
| C1 | - | 25.6 |
| C2 | - | 24.3 |
| C3 | - | 22.9 |
| C4 | - | 18.7 |

The present invention has been described with reference to various embodiments thereof. The foregoing detailed description and examples have been provided for clarity of understanding only, and no unnecessary limitations are to be understood therefrom. It will be apparent to those skilled in the art that many changes can be made to the described embodiments without departing from the scope of the invention defined by the appended claims. Thus, the scope of the invention should not be limited to the exact details of the compositions and structures described herein, but rather by the appended claims.

## Claims

1. A medicinal aerosol device (10) comprising a medicinal composition within a container (30) equipped with a valve (12) wherein the device comprises at least one sealing member (16), wherein the medicinal composition comprises a hydrofluoroalkane propellant **characterised in that** the at last one sealing member comprises a crosslinked perfluorinated elastomer.

2. A device according to claim 1 wherein the hydrofluoroalkane is selected from the group consisting of HFC-134a, HFC-227, and mixtures thereof.

3. A device as claimed in any preceding claim wherein the medicinal composition comprises a polar cosolvent.

4. A device according to claim 3 wherein the polar cosolvent is ethanol.

5. A device as claimed in any preceding claim wherein the sealing member is a valve seal.

6. A device as claimed in any preceding claim wherein the valve comprises a valve stem and the sealing member allows reciprocal movement of the valve stem.

7. A device as claimed in any preceding claim wherein the perfluorinated elastomer comprises interpolymerized units derived from monomers selected from the group consisting of perfluoro(alkylvinyl) ethers and perfluoro(alkoxyvinyl) ethers and mixtures thereof.

8. A device according to claim 7 wherein the perfluorinated elastomer further comprises interpolymerized units derived from tetrafluoroethylene.

9. A device as claimed in any preceding claim wherein the perfluorinated elastomer comprises a fluoropolymer having interpolymerized units derived from a nitrogen-containing cure site monomer.

10. A device as claimed in any preceding claim wherein the sealing member further comprises a filler.

11. A device as claimed in claim 10 wherein the filler is selected from the group consisting of perfluoroalkoxy resins, barium sulfate, magnesium oxide, and calcium.

12. The device according to claim 1 wherein the at least one sealing member is a diaphragm and the device comprises a valve stem, walls defining a diaphragm aperture, and a casing member having walls defining a formulation chamber and a casing aperture, wherein the valve stem passes through the diaphragm aperture and the casing aperture and is in slidable sealing engagement with the diaphragm aperture, and wherein the diaphragm is in sealing engagement with the casing member, the device having contained in the formulation chamber thereof a medicinal aerosol formulation.

13. A device as claimed in claim 12 wherein the medicinal composition comprises propellant selected from the group consisting of HFC-134a, HFC-227, and mixtures thereof.

## Patentansprüche

1. Medizinische Aerosolvorrichtung (10), die eine medizinische Zusammensetzung innerhalb eines Behälters (30) umfasst, der mit einem Ventil (12) ausgestattet ist, wobei die Vorrichtung mindestens ein Dichtungselement (16) umfasst, wobei die medizinische Zusammensetzung ein Hydrofluoralkan als Treibmittel umfasst, **dadurch gekennzeichnet, dass** das mindestens eine Dichtungselement ein quervernetztes perfluoriertes Elastomer umfasst.

2. Vorrichtung nach Anspruch 1, wobei das Hydrofluoralkan ausgewählt ist aus der Gruppe, die aus HFC-134a, HFC-227 und Mischungen daraus besteht.

3. Vorrichtung nach einem der vorhergehenden Ansprüche, wobei die medizinische Zusammensetzung ein polares Hilfslösemittel umfasst.

4. Vorrichtung nach Anspruch 3, wobei das polare Hilfslösemittel Ethanol ist.

5. Vorrichtung nach einem der vorhergehenden Ansprüche, wobei das Dichtungselement eine Ventildichtung ist.

6. Vorrichtung nach einem der vorhergehenden Ansprüche, wobei das Ventil einen Ventilschaft umfasst und das Dichtungselement die entgegengesetzte Bewegung des Ventilschaftes gestattet.

7. Vorrichtung nach einem der vorhergehenden Ansprüche, wobei das perfluorierte Elastomer interpolymerisierte Einheiten umfasst, die von Monomeren abgeleitet sind, welche aus der Gruppe ausgewählt sind, die aus Perfluor(alkylvinyl)ether und Perfluor(alkoxyvinyl)ether und Mischungen daraus besteht.

8. Vorrichtung nach Anspruch 7, wobei das perfluorierte Elastomer ferner interpolymerisierte Einheiten umfasst, die von Tetrafluorethylen abgeleitet sind.

9. Vorrichtung nach einem der vorhergehenden Ansprüche, wobei das perfluorierte Elastomer ein Fluorpolymer mit interpolymerisierten Einheiten umfasst, die aus einem Stickstoff enthaltenden Vernetzungsmonomer abgeleitet sind.

10. Vorrichtung nach einem der vorhergehenden Ansprüche, wobei das Dichtungselement ferner einen Füllstoff umfasst.

11. Vorrichtung nach Anspruch 10, wobei der Füllstoff ausgewählt ist aus der Gruppe, die aus Perfluoralkoxy-Harzen, Bariumsulfat, Magnesiumoxid und Calcium besteht.

12. Vorrichtung nach Anspruch 1, wobei das mindestens eine Dichtungselement ein Diaphragma ist und die Vorrichtung einen Ventilschaft, Wände, die eine Diaphragma-Öffnung festlegen, und ein Gehäuseelement umfasst, mit Wänden, die eine Formulierungskammer und eine Gehäuseöffnung festlegen, wobei der Ventilschaft durch die Diaphragma-Öffnung und die Gehäuseöffnung hindurchgeht und in gleitendem Dichtungseingriff mit der Diaphragma-Öffnung steht und wobei das Diaphragma in Dichtungseingriff mit dem Gehäuseelement steht, wobei die Vorrichtung in der Formulierungskammer davon eine medizinische Aerosolformulierung enthält.

13. Vorrichtung nach Anspruch 12, wobei die medizinische Zusammensetzung ein Treibmittel umfasst, welches ausgewählt ist aus der Gruppe, die aus HFC-134a, HFC-227 und Mischungen daraus besteht.

## Revendications

1. Dispositif aérosol pour médicament (10) comprenant une composition médicinale à l'intérieur d'un contenant (30) équipé d'une soupape (12), le dispositif comprenant au moins un élément d'étanchéité (16), et la composition médicinale comprenant un propulseur hydrofluoroalcane, **caractérisé en ce que** ledit au moins un élément d'étanchéité comprend un élastomère perfluoré réticulé.

2. Dispositif selon la revendication 1, dans lequel l'hydrofluoroalcane est sélectionné dans le groupe constitué de HFC-134a, de HFC-227, et de mélanges de ceux-ci.

3. Dispositif selon l'une quelconque des revendications précédentes, dans lequel la composition médicinale comprend un cosolvant polaire.

4. Dispositif selon la revendication 3, dans lequel le cosolvant polaire est l'éthanol.

5. Dispositif selon l'une quelconque des revendications précédentes, dans lequel l'élément d'étanchéité est un joint d'étanchéité de soupape.

6. Dispositif selon l'une quelconque des revendications précédentes, dans lequel la soupape comprend une tige de soupape et l'élément d'étanchéité permet un mouvement de va-et-vient de la tige de soupape.

7. Dispositif selon l'une quelconque des revendications précédentes, dans lequel l'élastomère perfluoré comprend des unités interpolymérisées dérivées de monomères sélectionnés dans le groupe constitué d'éthers perfluoro(alkylvinyliques) et d'éthers perfluoro(alcoxyvinyliques) et de mélanges de ceux-ci.

8. Dispositif selon la revendication 7, dans lequel l'élastomère perfluoré comprend en outre des unités interpolymérisées dérivées de tétrafluoroéthylène.

9. Dispositif selon l'une quelconque des revendications précédentes, dans lequel l'élastomère perfluoré comprend un fluoropolymère comportant des unités interpolymérisées dérivées d'un monomère apportant un site de durcissement contenant des atomes d'azote.

10. Dispositif selon l'une quelconque des revendications précédentes, dans lequel l'élément d'étanchéité comprend en outre une charge.

11. Dispositif selon la revendication 10, dans lequel la charge est sélectionnée dans le groupe constitué des résines perfluoroalcoxydes, du sulfate de baryum, de l'oxyde de magnésium, et du calcium.

12. Dispositif selon la revendication 1, dans lequel ledit au moins un élément d'étanchéité est un diaphragme et le dispositif comprend une tige de soupape, des parois délimitant une ouverture de diaphragme, et un élément sous forme de boîtier comportant des parois délimitant une chambre de formulation et une ouverture de boîtier, la tige de soupape passant dans l'ouverture de diaphragme et dans l'ouverture de boîtier et coulissant de façon étanche dans l'ouverture de diaphragme, et le diaphragme étant en contact étanche avec l'élément sous forme de boîtier, la chambre de formulation du dispositif contenant une composition médicinale aérosol.

13. Dispositif selon la revendication 12, dans lequel la composition médicinale comprend un propulseur sélectionné dans le groupe constitué de HFC-134a, de HFC-227, et de mélanges de ceux-ci.
